# FASCICULE DE BREVET EUROPEEN

(11) **EP 2 017 290 B1**
(45) Date de publication et mention de la délivrance du brevet: **07.09.2016**
(21) Numéro de dépôt: 08160503.2
(22) Date de dépôt: 16.07.2008
(51) Int. Cl.: A61K 8/73, A61Q 19/08, A61K 36/07, A61K 8/97, C08B 37/00

(54) **Extrait polysaccharidique de Lentinus et compositions pharmaceutiques, cosmétiques ou nutraceutiques comprenant un tel extrait.**
Polysaccharidextrakt aus Lentinus und pharmazeutische, kosmetische oder nahrungsergänzende Zusammensetzungen, die dieses Extrakt enthalten
Polysaccharide extract of Lentinus and pharmaceutical, cosmetic or nutraceutical compositions comprising such an extract.

(30) Priorité: 20.07.2007 FR 0756641
(43) Date de publication de la demande: 21.01.2009
(73) Titulaire: CASTER, 75008 Paris (FR)
(72) Inventeur: Ales, Patrick, 75008, Paris (FR); Escaut, Alexandre, 92100, Boulogne (FR); Choulot, Jean-Christophe, 78120, Rambouillet (FR)
(74) Mandataire: Dossmann, Gérard

(56) Documents cités:
- EP-A- 1 389 466
- EP-A- 1 495 753
- FR-A- 2 829 389
- JP-A- 62 142 120
- KR-A- 20060 129 630
- US-A- 5 997 875
- PRIETO J M ET AL: "INFLUENCE OF TRADITIONAL CHINESE ANTI-INFLAMMATORY MEDICINAL PLANTS ON LEUKOCYTE AND PLATELET FUNCTIONS" JOURNAL OF PHARMACY AND PHARMACOLOGY, LONDON, GB, vol. 55, no. 9, 1 septembre 2003 (2003-09-01), pages 1275-1282, XP009023482 ISSN: 0022-3573
- ZHENG R ET AL: "Characterization and immunomodulating activities of polysaccharide from Lentinus edodes" INTERNATIONAL IMMUNOPHARMACOLOGY, ELSEVIER, AMSTERDAM, NL, vol. 5, no. 5, mai 2005 (2005-05), pages 811-820, XP004792733 ISSN: 1567-5769

## Description

La présente invention concerne un extrait polysaccharidique issu de champignon du genre Lentinus ainsi que le procédé de préparation d'un tel extrait. Cet extrait riche en polysaccharides est utilisable en tant que préparation nutraceutique, cosmétique ou pharmaceutique.

La peau est un organe à part entière, jouant un rôle fondamental dans la santé et également dans l'apparence. Barrière à la fois résistante et fragile, elle est en perpétuel renouvellement. Malgré cela, les premiers signes du vieillissement cutané se manifestent, discrètement, avant 30 ans. Avec le temps, la peau perd sa souplesse et sa capacité à retenir l'eau diminue. Bien que la déshydratation ne soit qu'un des aspects du vieillissement cutané, il apparaît que la teneur en eau est étroitement liée à nombre de caractéristiques morphologiques et moléculaires de la peau normale.

La protection contre la perte en eau est principalement assurée par l'épiderme et sa couche cornée (Verdier-Sevrain., S. & F., Bonte, 2007, J. Cosmet. Dermatol. 6(2):pp 75-82). L'épiderme est un épithélium pavimenteux stratifié constitué principalement de kératinocytes (90 %), le derme étant le support conjonctif nourricier de l'épiderme dont la principale finalité biologique est la formation de la couche cornée. Le «stratum corneum» ou couche cornée est la zone la plus superficielle de l'épiderme. Elle est constituée d'un empilement de cellules plates anucléées, les cornéocytes, résultat ultime du processus de différenciation et de prolifération des kératinocytes. Le stratum corneum, par sa solidité et grâce à sa structure stratifiée compacte, assure une fonction barrière : il s'oppose à la perte en eau transcutanée et protège les couches sous-jacentes des agressions mécaniques, chimiques et de l'irradiation ultraviolette.

La teneur en eau de la peau est conditionnée par des différents paramètres parmi lesquels se trouve, entre autres, le degré d'élimination d'eau, qui dépend de l'équilibre existant entre la capacité de la peau à retenir l'eau et les pertes transépidermiques par évaporation, sorte de déshydratation physiologique reflétant l'intégrité de la barrière épithéliale cutanée ainsi que sa fonctionnalité.

Tous les épithéliums, compris celui de la peau, sont constitués de cellules associées les unes aux autres avec plus ou moins de cohésion. Les jonctions serrées ou imperméables (tight junctions) sont spécifiques de tous les tissus épithéliaux. Localisées généralement au sommet des cellules, elles scellent les cellules épithéliales cutanées entre elles et empêchent ainsi le passage des fluides par la voie intercellulaire à l'extérieur des tissus. Leur principale fonction est donc d'assurer l'étanchéité de l'épiderme et d'éviter que l'organisme ne se vide de son eau au contact du milieu extérieur (Tsuruta, D., et al., 2002, Trends Cell Biol. 12(8): pp 355-7).

La jonction entre des cellules de l'épiderme est due en effet à des interactions homophiles qui concernent plusieurs protéines d'adhérence. La première est l'occludine (du latin occludere = enfermer). C'est une protéine transmembranaire d'un poids de 64 kDa, constituée d'une chaîne polypeptidique qui traverse quatre fois la membrane. La deuxième est la claudine (du latin claudere = fermer), une protéine transmembranaire d'un poids de 22 kDa, constituée également d'une chaîne polypeptidique qui traverse quatre fois la membrane. La claudine fait partie d'une famille d'au moins 24 membres, dont plusieurs peuvent être présents dans une même jonction. La troisième protéine est JAM (junctional adhesion molecule), une protéine d'un poids moléculaire de 33 kDa et dont la chaîne polypeptidique traverse la membrane une seule fois.

Occludine, claudines et JAM s'assemblent pour former un réseau de fibrilles qui ceinture le domaine apical des cellules épithéliales. Les jonctions serrées sont liées à des protéines intracellulaires comme ZO-1 et ZO-2 (zonula occludens) qui à leur tour sont liées au cytosquelette (actine). Cette interaction avec le cytosquelette détermine la localisation de la jonction au domaine apical de la cellule.

En fonction de l'expression des protéines qui constituent les jonctions serrées, les cellules de l'épiderme sont plus ou moins capables de limiter la perméabilité de l'épithélium cutané. L'hydratation de la peau reste un phénomène complexe et d'une importance capitale en cosmétologie ou en dermatologie d'où la recherche constante de nouveaux soins qui reprogramment le processus d'hydratation en profondeur et en surface pour regonfler la peau de l'intérieur et lisser le relief cutané.

Lentinus edodes (Shii-take), un champignon forestier comestible du Japon, est connu dans de nombreux pays asiatiques (Chine, Corée, etc.) comme un des meilleurs champignons pour son goût et son parfum. Le Lentinus edodes est en effet caractérisé par quelques substances spécifiques, comme, par exemple, la guanosine 5'-monophosphate qui induit un parfum agréable et une substance aromatique, la lenthionine.

Il existe actuellement au monde des centaines de champignons comestibles mais la majorité n'est pas cultivée. Seulement quelques champignons Agaricus bisporus en Europe et Lentinus edodes en Asie sont cultivés à grande échelle, mais Agaricus bisporus est cultivé sur compost et utilisé frais alors que le Lentinus est cultivé sur bois et, souvent séché. Le séchage du Lentinus permet en effet de le conserver, tout en préservant son arôme et son goût.

Le Shii-take est un champignon cher qui est connu aussi sous le nom de « Elixir de la vie ». D'après le grand médecin chinois WU SHUI qui a vécu au temps de la dynastie Ming (1364-1644), le shii-take augmente la vigueur et la vitalité et est très efficace dans le traitement préventif de l'hémorragie cérébrale. Récemment, les propriétés médicales du Shii-take ont été étudiées par des chercheurs japonais et on a pu mettre en évidence des actions antivirale, antibiotique, antitumorale et hypolipidémique.

Il y a environ 800 ans, on a commencé à cultiver le champignon en Chine (le nom chinois du Shii-take est HOANG-KO). Aujourd'hui il existe près de 230000 petits producteurs de Shii-take au Japon. Ce champignon est cultivé également en Corée et en Chine et est cueilli à l'état sauvage dans les forêts des autres pays asiatiques. En 1974, la production totale du Japon a été de 12000 de tonnes de champignons séchés et de 55000 de tonnes de champignons frais. Le Shii-take est exporté uniquement séché.

Il est connu du brevet FR2776184 un extrait aqueux de Lentinus riche en lentinane, pour ses propriétés anti-tumorales. Le brevet FR2829389 décrit un procédé d'extraction de Lentinus comprenant des étapes d'hydrolyse enzymatique afin d'obtenir un extrait capable d'inhiber l'activité de certaines métalloprotéases, les MMPs et en particulier les MMP-1 et 2.

La composition chimique du Shii-take a été déterminée par des méthodes standard d'analyse publiées par l'Association de Chimie Analytique. Les dosages ont porté sur l'humidité, les protéines brutes, les graisses et les cendres. Les protéines brutes ont été déterminées par rapport à l'azote contenu dosé par la méthode de Kjeldahl, en utilisant le facteur de conversion (N x 6,25). En fait, les études et les dosages faits sur la protéine brute ont montré que seulement une partie est digestible. D'autres travaux démontrent que le taux probable de digestibilité est de 60-70%. Ce taux réduit s'explique par le fait que les champignons contiennent un pourcentage non négligeable d'azote non protéique lié à la chitine des parois cellulaires. On peut donc considérer que le facteur de conversion est 70% N x 6,25 ou (N x 4,38).

Le pourcentage de graisse contenu dans les champignons peut varier entre 1 et 15-20% du poids sec. En moyenne, les champignons contiennent 2 à 8% de graisses et on y trouve toutes les catégories de lipides comme les acides gras libres (stéarique, oléique, palmitique et linoléique) les mono, di et triglycérides, les stérols (ergostérol) stérols-esters et phospholipides. Les graisses ont été analysées par extraction aux solvants ; les fibres, comme résidu après digestion, les cendres comme résidu après incinération. Les champignons frais contiennent entre 3 et 28% d'hydrates de carbone et 3 à 32% de fibres. Les carbohydrates ont été calculés par différence, comme carbohydrates totaux (fibres incluses) ou comme carbohydrates sans fibres. On peut penser que la plus grande quantité d'azote non protéique se trouve sous forme de chitine et, en petite quantité sous forme d'urée ou de sels d'ammonium.

| Echantillon | Humidité | Protéine Brute N x 4,68 | Graisse | Carbohydrates | | | Cendres | Valeur Energéti que Kcal |
|---|---|---|---|---|---|---|---|---|
| | | | | Total | Sans N | Fibres | | |
| Séché (1) | 15,8 | 10,3 | 1,9 | 2,3 | 5,8 | 6,5 | 5,5 | 375 |
| Séché (4) | 19,5 | 26,0 | 2,9 | 5,0 | 1 ,5 | 13,5 | 6,1 | 345 |
| Séché (3, 4) | 18,4 | 13,1 | 1,2 | 9,2 | 4,5 | 14,7 | 6,5 | 333 |
| Frais (3, 4) | 90,0 | 17,5 | 8,0 | 7,5 | 9,5 | 8,0 | 7,0 | 387 |
| Frais (2) | 91 ,8 | 13,4 | 4,9 | 8,0 | 0,7 | 7,3 | 3,7 | 392 |

| | | | | | | | | |
|---|---|---|---|---|---|---|---|---|
| 1 - Singer (1961) 2 - Organisation Agro-Alimentaire (1972) 3 - Soweda (1965) ; 4 - Adriano et Gruz (1933) | | | | | | | | |

Le Lentinus edodes contient 17.5 % de son poids sec en protéines et pratiquement tous les acides aminés essentiels. Environ 25-35% des acides aminés totaux sont des acides aminés libres, le reste étant lié aux protéines. En dehors des acides aminés communs, on a détecté dans ces champignons des acides aminés rares ou des substances azotées telle que : β-alanine, sulfoxyde de méthionine, acide cystéique, hydroxyproline, hydroxylysine, acide α-aminoadipique, acide α, β et y aminobutyrique, acide pipecolique et 5-hydroxypipecolique, phosphosérine, cystathione canavanine, créatinine, citrulline, arnithine, glucosamine et éthanolamine.

**Composition en acides aminés de Lentinus edodes selon Kagawa (1970), Sawada (1965), Sugimosi et col. (1971).**

| Acide aminé | mg d'acides aminés par gramme de protéine brute. |
|---|---|
| Isoleucine | 218 |
| Leucine | 348 |
| Lysine | 174 |
| Méthionine | 87 |
| Phénylalanine | 261 |
| Cvstine | non déterminé |
| Tvrosine | 174 |
| Thréonine | 261 |
| Tryptophane | non déterminé |
| Valine | 261 |
| Arginine | 348 |
| Histidine | 87 |
| Alanine | 305 |
| Acide aspartique | 392 |
| Acide glutamique | 1182 |
| Glycine | 218 |
| Sérine | 261 |
| Total des acides aminés | 4962 |

On trouve dans les champignons du genre Lentinus des pentoses (xylose et ribose), méthyl pentoses (rhamnose et fucose) hexoses (glucose, galactose et mannose), disaccharides (sucrose), sucres aminés (glucosamine et N-acétylglucosamine), sucres-alcools (manutol et inositol) sucres acides (acide galacturonique et glucuronique) etc. Le mannitol se trouve en concentration élevée (9-13%).

Le champignon Lentinus contient des polysaccharides et de la chitine (polymère de N-acétylglucose amine). De même, on trouve du α, a' tréhaloxe, nommé de façon populaire, le sucre des champignons.

Les vitamines les plus répandues dans le champignon Shii-take sont la thiamine, la riboflavine, la niacine, la biotine. La vitamine A (rétinol) ne se trouve pas de façon habituelle dans ces champignons mais on peut doser parfois la provitamine A exprimée en équivalents de β-carotène. De même, la vitamine D est rare mais on trouve de l'ergostérol qui peut être transformé en vitamine D par irradiation ultraviolette.

La demanderesse a mis au point de nouveaux extraits polysaccharidiques issus de champignons Lentinus **edodes,** qui constituent l'objet de l'invention.

L'invention a également pour objet le procédé d'obtention de cet extrait.

Un autre objet est constitué par les applications de cet extrait comme composition nutraceutique, cosmétique ou comme médicament.

D'autres objets apparaîtront à la lecture de la description, des dessins et des exemples qui suivent.
Les figures 1 et 2 montrent une analyse par spectrométrie de masse (MALDI-TOF) de l'extrait polysaccharidique obtenu dans l'exemple 1. Les poids moléculaires des composés visualisés s'étagent de 500 à 10 000 m/z (masse/charge).
Les figures 3 et 4 montrent des analyses chromatographiques de l'extrait polysaccharidique obtenu dans l'exemple 1. L'analyse résumée sur la figure 3 a été réalisée sur chromatographie liquide échangeuse d'ions. Celle de la figure 4 est une chromatographie d'exclusion stérique.
Les figures 5a et 5b, 6a et 6b, 7a et 7b montrent l'expression des protéines structurales de jonctions serrées dans des cellules HaCaT par une technique d'immunohistochimie. Les figures 5a et 5b montrent la détection de claudine, les figures 6a et 6b, la détection d'occludine-1 et les figures 7a et 7b, la détection de ZO-1. Les figures 5a, 6a et 7a sont les témoins négatifs des expériences d'immuno-marquage effectuées. Les figures 5b, 6b et 7b sont le résultat des expériences en présence de l'extrait polysaccharidique selon l'invention dilué à 0,1% dans du milieu de culture cellulaire.

L'extrait polysaccharidique issu de champignon Lentinus **edodes,** conforme à l'invention, comprend des monosaccharides, des polysaccharides, et en poids, de 2 à 8% de tréhalose, de 1 à 4 % de glucose et de 30 à 60 % d'alditols par rapport au poids total de l'extrait sec.

De préférence, cet extrait comprend, outre des monosaccharides et des polysaccharides, de 2 à 6 % de tréhalose, de 2 à 3 % de glucose et de 45 à 55 % d'alditols en poids par rapport au poids total de l'extrait sec. Plus préférentiellement, cet extrait comprend outre des monosaccharides et des polysaccharides, de 3 à 5 % de tréhalose, de 2 à 3 % de glucose et de 45 à 50 % d'alditols en poids par rapport au poids total de l'extrait sec.

De préférence, les alditols contenus dans cet extrait selon l'invention comprennent de l'arabinitol et du mannitol.

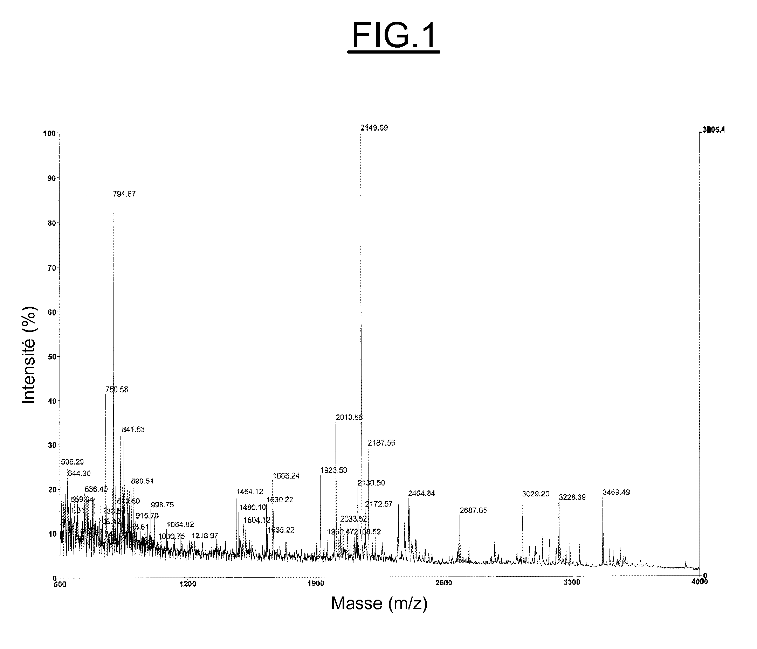

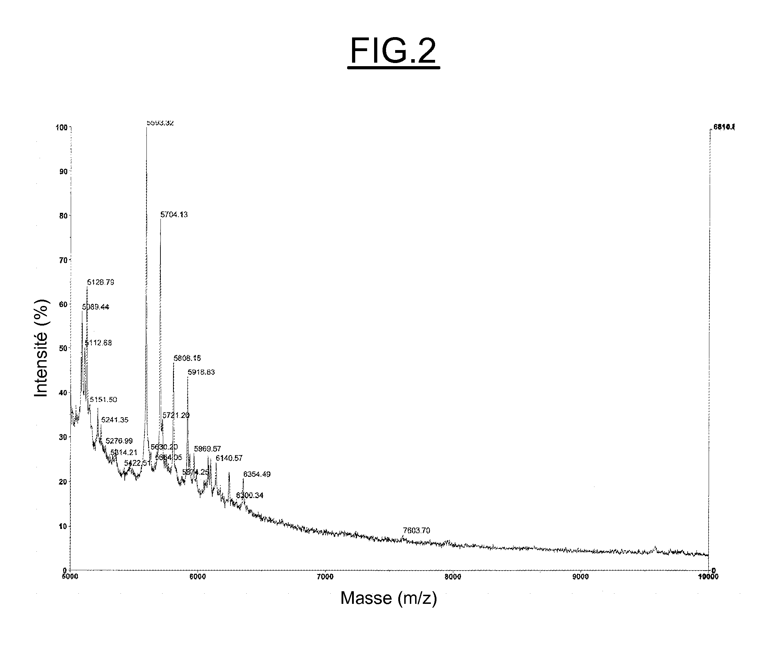

Un extrait polysaccharidique particulièrement préféré est défini par les analyses de spectrométrie de masse des figures 1 et 2.

L'extrait selon l'invention peut se présenter sous forme de poudre.

Le procédé d'extraction permettant l'extraction sélective d'un extrait polysaccharidique issu de Lentinus **edodes** selon l'invention est caractérisé par le fait que l'on procède à une ou plusieurs extractions solide/liquide, l'extrait liquide est précipité par un solvant organique, centrifugé, filtré, puis le précipité est ensuite séché pour obtenir l'extrait polysaccharidique.

**L**a ou les extractions s'effectuent à l'eau bouillante **et** le solvant organique utilisé est de l'éthanol.

Les extraits sont obtenus à partir du procédé précédemment décrit à partir de champignons Lentinus **edodes** qui peuvent être utilisés séchés. De façon générale, les champignons frais contiennent 85 à 95% d'eau et les champignons séchés à l'air, entre 5 et 20% d'eau. **L**es champignons sont des Lentinus edodes autrement nommés lentinula edodes, lentin du chêne, Shii-take, Shiitake, Cortinellus Shii-take ou Cortinellus edodes. Le nom de Cortinellus Shii-take a été le nom le plus couramment utilisé mais Singer (1941) a remis en question l'appartenance du Shii-take au genre Cortinellus. Depuis, on utilise comme nom scientifique correct Lentinus edodes (Berk) Sing. De même, dans l'ancien système de classification, le Shii-take appartenait à la famille des Agaricacées, alors que le système moderne de classification place le Lentinus edodes dans la famille des Tricholomatacées.

L'extrait polysaccharidique selon l'invention agit sur l'expression des protéines de cohésion appelées jonctions serrées ou imperméables (tight junctions), notamment l'occludine, la claudine et la JAM (junctional adhesion molecule).

L'invention a également pour objet des compositions pharmaceutique, cosmétique ou nutraceutique comprenant un tel extrait polysaccharidique, et éventuellement un véhicule inerte physiologiquement acceptable, pour le traitement d'humains ou de mammifères souffrant d'une condition ou d'une maladie liée à l'absence ou à une dégradation excessive ou pathologique des protéines de cohésion.

L'invention est ainsi relative à des compositions pharmaceutiques comprenant un tel extrait polysaccharidique pour traiter les dermatoses telles que le psoriasis, la dermatite atopique, l'ichtyose.

L'invention est également relative à des compositions cosmétiques ou nutraceutiques comprenant un tel extrait polysaccharidique et éventuellement un véhicule inerte physiologiquement ou cosmétiquement acceptable pour le traitement des lésions de la peau et des muqueuses dues au vieillissement telles que les lésions provoquées par l'action du rayonnement solaire, le vieillissement actinique, les agressions extérieures comme les effets délétères du tabac, ou de la pollution par exemple, et les conséquences de ces agressions.

L'extrait polysaccharidique selon l'invention peut être incorporé ou formulé dans un véhicule polymérique ou un système de délivrance pour une utilisation topique ou locale, comme dans le cas du traitement d'une maladie parodontale, pour être délivré directement dans la poche parodontale.

De telles compositions peuvent être utilisées à titre à titre préventif ou curatif.

Les compositions selon l'invention peuvent être ingérées ou appliquées sur la peau ou surface cutanée d'un individu. Selon le mode d'administration, la composition selon l'invention peut se présenter sous toutes les formes habituellement utilisées en cosmétique, nutraceutique ou comme médicament. Ces compositions peuvent en particulier être formulées sous la forme de tablettes, de gélules, de capsules, de pommades, de crèmes, de laits, de gels.

Les exemples suivants illustrent l'invention sans la limiter aucunement.

### Exemple 1: Extraits totaux aqueux et précipité alcoolique

1 kilogramme de Lentinus edodes séché est broyé dans un broyeur à couteaux Henri. La poudre obtenue est extraite par 3 x 10 litres d'eau distillée bouillante. On agite 30 minutes à 90°C et on filtre sur un filtre Büchner de 50 litres sur une couche d'adjuvant de filtration (célite). On concentre à 5 litres dans un évaporateur à couche mince Luwa, puis on lyophilise dans un appareil à lyophiliser Serail. Une partie aliquote de chaque extrait aqueux est dissoute dans de l'eau distillée et précipitée par addition de 1,5 volume d'éthanol 96°. On laisse une nuit en chambre froide et on centrifuge (IEC Centra 7R). On sèche en dessiccateur sous vide.

### Exemple 2 : analyse de l'extrait selon l'invention par spectométrie de masse

L'extrait de l'exemple 1 a été analysé par spectrométrie de masse (MALDI-ToF). Le MALDI (Matrix Assisted Laser Desorption Ionization) est une méthode d'ionisation permettant d'analyser des molécules dont le poids moléculaire est supérieur à 1000 Da. L'analyseur couplé à la source MALDI est un analyseur à temps de vol (ToF, Time of Flight) qui est bien adapté à l'ionisation pulsée par désorption laser. La séparation dans un analyseur à temps de vol repose sur le fait que des ions de masse différente, accélérés à une énergie cinétique uniforme, ont des vitesses différentes, et donc un temps de vol différent pour parcourir une distance donnée.

Les figures 1 et 2 montrent une visualisation de l'ensemble des composées dont les poids moléculaires se situent entre 500 et 10000 m/z.

### Exemple 3 : Caractérisation de l'extrait de l'exemple 1 par chromatographie

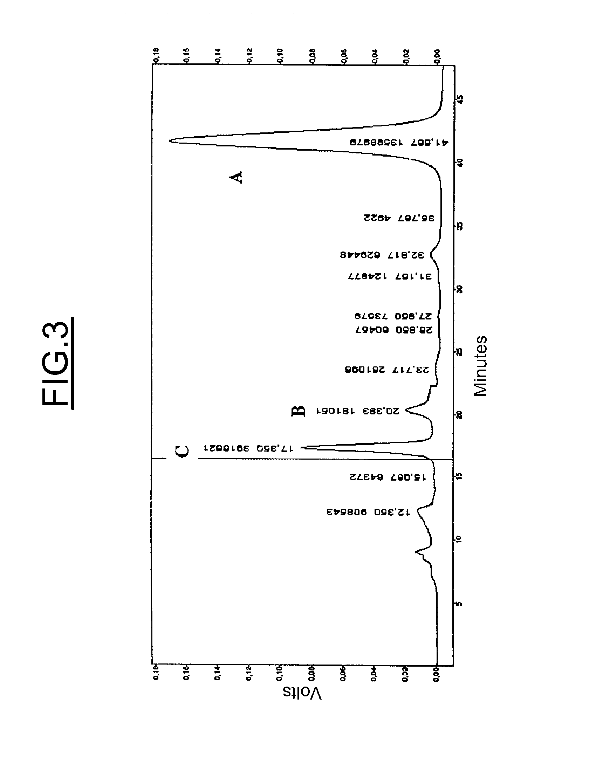

L'analyse de l'extrait brut de l'exemple 1 a été réalisée par la chromatographie liquide d'échange d'ions sur une colonne CARBO-Sep (CHO-682, Interchim) dont la sélectivité unique permet la séparation de mono- et disaccharides en utilisant seulement l'eau pure comme éluant. Le chromatogramme obtenu (Fig. 3) montre la présence majoritaire d'une petite molécule hydroxylée (A) et, entre autres, deux composés pouvant correspondre à un mono- (B) et un disaccharide (C).

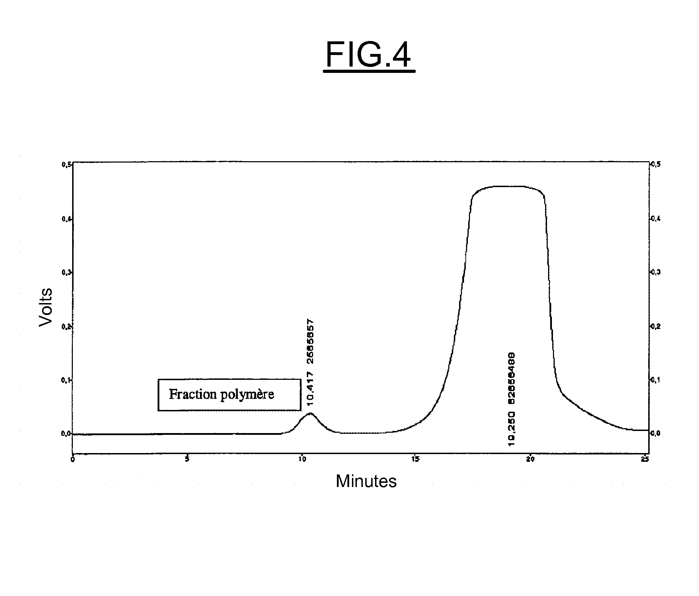

Dans un deuxième temps, l'analyse de l'extrait brut a été réalisée à l'aide de la chromatographie d'exclusion stérique sur la colonne Shodex OHpak B-804 (limite d'exclusion : 4 x 10⁵ kDalton). Le profil chromatographique obtenu montre que seulement 2% de l'extrait correspond à des polysaccharides (Fig. 4).

### Exemple 4 : Ultrafiltrations de l'extrait de l'exemple 1

Les ultrafiltrations successives de l'extrait brut réalisées sur les membranes d'ultrafiltration en cellulose (Diaflo^{®}-Amicon Co) caractérisées par trois seuils de coupure de 100 kDalton (kD), 10 kD et 1 kD ont conduit à l'obtention de 4 fractions (Lent. 100 = PM > 100 kD; Lent 10 = 10 kD < PM < 100 kD ; Lent 1 = 1 kD < PM < 10 kD et Lent. UF = PM < 1 kD) dont la teneur en poids est présentée ci-dessous.

Pour chaque étape, le lavage à l'eau distillé est considéré comme terminé lorsque 10 volumes de liquide ont traversé la membrane.

**Tableau 1.**

| Fractions | Lent. 100 | Lent. 10 | Lent. 1 | Lent. UF |
|---|---|---|---|---|
| Poids (mg) | 47 | 127 | 24 | 1874* |
| % | 2,4 | 6,5 | 1,2 | 89,9 |

| | | | | |
|---|---|---|---|---|
| * Après correction de la teneur en eau | | | | |

Les fractions contenant des polysaccharides, Lent. 100 et Lent. 10 correspondent à 8.9% (2.4% + 6.5%) du poids total de l'extrait actif.

Une hydrolyse acide totale de 4 ultrafiltrats suivie d'une analyse des sucres par chromatographie en phase liquide a été employée pour déterminer à nature des composants glucidiques. La fraction Lent. UF, le plus important composant de l'extrait (89.9%), contient à part des monosaccharides (glucose, fucose, arabinose, mannose, xylose, arabinitol, mannitol) et disaccharides (tréhalose), des acides aminés, vitamines et des minéraux.

La composition des fractions Lent. 1, Lent. 10 et Lent. 100 en sucres est présentée dans le Tableau 2.

**Tableau 2. Composition des fractions en sucres neutres**

| Sucres neutres | Rhamnose | Fucose | Ribose | Arabinose | Xylose | Mannose | Galactose | Glucose |
|---|---|---|---|---|---|---|---|---|
| Lent 1 | 12,3 | 10,4 | 5,2 | 2 | 2,6 | 20 | 32,5 | 15 |
| Lent 10 | 12,6 | 5,3 | 15,4 | 1,3 | 4,5 | 21 | 13,8 | 26,1 |
| Lent 100 | 4 | 5,1 | 7,1 | 5,4 | 4,6 | 17,1 | 26,4 | 29,3 |

| | | | | | | | | |
|---|---|---|---|---|---|---|---|---|
| * calculé sur la totalité des sucres neutres identifiés | | | | | | | | |

L'ensemble des résultats obtenus suite aux différentes analyses de l'extrait actif de l'exemple 1 est présenté dans le Tableau 3.

**Tableau 3.**

| Constituants | (Lent. 100 + Lent 10) dont polysaccharides | Fraction > 1KD | Autres constituants | Tréhalose | Glucose | Autres monosaccharides | Alditols |
|---|---|---|---|---|---|---|---|
| En % | 8,9 | 1,2 | 34,1 | 4 | 2,6 | 2,9 | 46,3 |

### Exemple 5 : Mise en évidence de l'action d'un extrait de Shii-take sur l'hydratation de la peau.

L'expression des protéines structurales des jonctions serrées (claudine, occludine, ZO-1) a été évaluée par la technique d'immunohistochimie dans des kératinocytes contrôles ou traités par l'extrait de shii-take.

Des kératinocytes humains HaCaT (German Cancer Research Center, Heidelberg, Germany), ont été cultivés à l'étuve (37° C, 5% CO₂) en milieu DMEM Glutamax (Invitrogen, GIBCO, cat n° 61965) avec 10 % FCS. A J₀, 8.10³ cellules sont ensemencées sur lames Lab-Tek (VWR International S.A.S., Nalge Nunc International, cat n° 177445), puis remises à l'étuve. A J₃, le milieu de culture est remplacé par l'extrait de shii-také selon l'exemple 1 dilué à 0,1% dans du milieu de culture puis les lames sont remises à l'étuve.

Après 16h d'incubation en présence de l'extrait testé, les cellules sont fixées au formaldhéyde 4%, et perméabilisées au Triton 0.1%. Les lames sont incubées ensuite 2h à température ambiante en présence de l'un des anticorps primaires marqueurs des jonctions serrées :
* Anticorps lapin anti-occludine (Invitrogen, Zymed laboratories, cat. N° 71-1500) dilution 1:35, dans du PBS contenant 1% de BSA
* Anticorps lapin anti-claudine-1 (Invitrogen Zymed laboratories, cat. N° 51-9000) dilution 1:35, dans du PBS contenant 1% de BSA
* Anticorps lapin anti-ZO-1 (Invitrogen Zymed laboratories, cat. N° 61-7300) dilution 1:90, dans du PBS contenant 1% de BSA

Après lavage, une seconde incubation de 2h est réalisée avec une solution de PBS avec 1% de BSA contenant un anticorps chèvre anti-lapin conjugué à l'AlexaFluor 488 (Invitrogen, Molecular Probes, cat n° A11008) dilution 1:200, pour le marquage des anticorps primaires.

Les lames sont rincées, puis montées (Fluorescent Mounting Medium, Dako, cat n° S3023) et observées au microscope Nikon TE2000E, avec un objectif 100X.

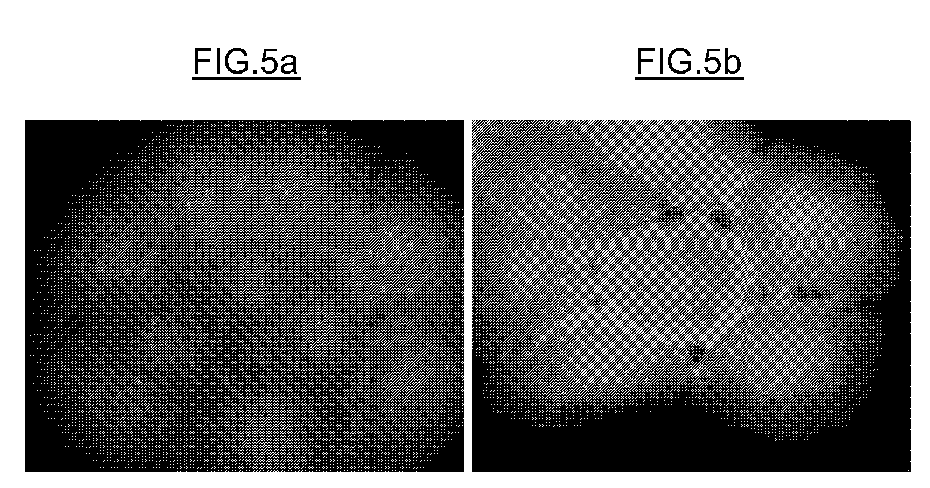

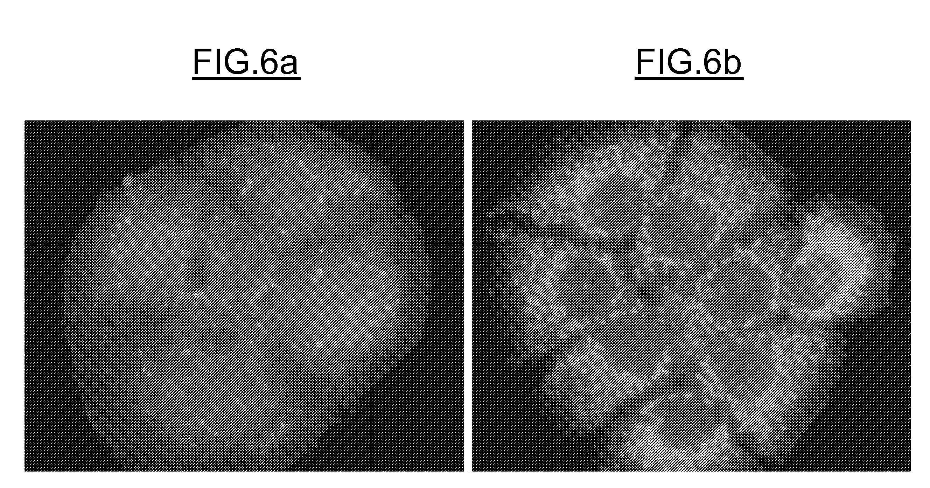

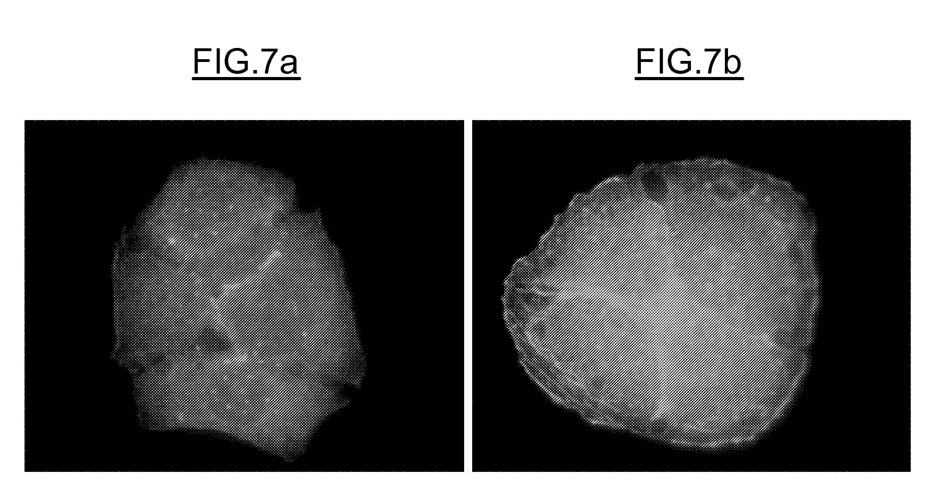

Les figures 5, 6 et 7 montrent que le traitement des kératinocytes avec l'extrait polysaccharidique éthanolique de shii-take induit une augmentation significative de l'expression des trois protéines étudiées, claudine, occludine et ZO-1. Or, il est connu que les modifications de l'expression de ces protéines indispensables à la cohésion des cellules de l'épiderme conduisent aux modifications de l'hydratation de la peau (Hadj-Rabia et al., 2004, Gastroenterology, 127(5):pp 1386-90).

Ces résultats montrent donc que le traitement de cellules avec l'extrait polysaccharidique à 0.1 % contribue à une meilleure hydratation de l'épiderme.

**Exemple 6 : Composition nutraceutique anti-âge à base de lentinus :**

| | |
|---|---|
| Vitamine C | 30 mg |
| Vitamine E | 10 mg |
| Sélénium | 75 µg |
| Extrait de thé vert | 60 mg |
| Huile d'onagre | 120 mg |
| Huile de bourrache | 120 mg |
| Huile de pépins de raisin | 120 mg |
| Extrait de houblon | 60 mg |
| Extrait de lentinus selon l'exemple 1 | 1 à 50 mg |

| | |
|---|---|
| Dans les compositions nutraceutiques des exemples 6, 7 et 8, l'extrait de lentinus peut être utilisé seul ou en composition avec d'autres actifs (Acides gras, minéraux, vitamines) dans des gélules ou des capsules. | |

**Exemple 7 : Composition nutraceutique pour l'éclat du teint**

| | |
|---|---|
| Lactobacillus lactis | 2 milliards de bactéries |
| Bifidobacterium lactis | 2 milliards de bactéries |
| Extrait de céramide de blé | 20 mg |
| Extrait de lentinus selon l'exemple 1 | 1 à 50 mg |

**Exemple 8 : Composition nutraceutique activatrice de hâle**

| | |
|---|---|
| Concentré de caroténoides | 25 mg |
| Vitamine E | 10 mg |
| Sélénium | 75 µg |
| Concentré de lycopène | 16 mg |
| Concentré de lutéine | 5 mg |
| Huile d'olive | 50 mg |
| Huile de bourrache | 100 mg |
| Extrait de lentinus selon l'exemple 1 | 1 à 50 mg |

**Exemple 9 : Composition cosmétique à base de lentinus pour "shampooing Energie"**

| INGREDIENTS | % |
|---|---|
| AQUA | QSP 100 |
| ROSMARINUS OFFICINALIS EXTRACT | 20 |
| SALVIA OFFICINALIS EXTRACT | 20 |
| SODIUM LAURETH SULFATE | 8 |
| COCAMIDOPROPYL HYDROXYSULTAINE | 3 |
| PEG-4 RAPESEEDAMIDE | 2 |
| LAURYL GLUCOSIDE | 1 |
| SODIUM LAUROYL SARCOSINATE | 1 |
| PEG-200 HYDROGENATED GLYCERYL PALMATE | 0,6 |
| GLYCERINE | 0,4 |
| PARFUM | QS |
| PANAX GINSENG EXTRACT | 0,35 |
| LACTITOL | 0,3 |
| XYLITOL | 0,3 |
| PEG-7 GLYCERYL COCOATE | 0,25 |
| CONSERVATEURS | QS |
| PEG-40 HYDROGENATED CASTOR OIL | 0,19 |
| ALCOOL DENAT. | 0,15 |
| LENTINUS EDODES | 0,02 à 5 |
| ZANTHOXYLUM ALATUM | 0,01 |

**Exemple 10 : Composition cosmétique à base de lentinus pour lotion capillaire**

| INGREDIENTS | % |
|---|---|
| AQUA | QSP 100 |
| ALCOOL DENAT. | 29 |
| GLYCERINE | 6 |
| CANANGA ODORATA OIL | 1,2 |
| CITRUS MEDICA LIMONUM OIL | 1,2 |
| PARFUM | 1 |
| MELALEUCA LEUCADENDRON | 0,6 |
| ROSMARINUS OFFICINALIS OIL | 0,6 |
| SALVIA OFFICINALIS OIL | 0,6 |
| CUPRESSUS SEMPERVIRENS | 0,45 |
| SERENOA SERRULATA | 0,2 |
| CONSERVATEURS | QS |
| VITIS VINIFERA SEED EXTRACT | 0,1 |
| SODIUM MANNURONATE METHYLSILANOL | 0,06 |
| LENTINUS EDODES | 0,025 à 5 |
| ACETYL TETRAPEPTIDE-2 | 0,0001 |

**Exemple 11 : Composition cosmétique à base de lentinus pour un lait pour le corps**

| Phase | Matière première / Nom commercial | % |
|---|---|---|
| A | EAU OSMOSEE | QSP 100 |
| | GLYCERINE | 5,0 |
| | | |
| | HUILE DE VASELINE FLUIDE | 5,0 |
| B1 | DIISOPROPYL SEBACATE | 5,0 |
| | PLUROL DIIS0STEARIQUE | 0,5 |
| | CONSERVATEUR | QSP |
| | | |
| B2 | ACRYLATES/C10-30 ALKYL ACRYLATE CROSSPOLYMER | 0,15 |
| | | |
| B3 | PALMOTENE | 0,5 |
| C | TROMETHAMINE A 20 % | 0,9 |
| | | |
| D | ACIDE CITRIQUE (SOLUTION A 10%) | 0,1 |
| | | |
| E | GOMME XANTHANE | 0,2 |
| | | |
| F | SOLUTION TAMPON pH 7.4 | QSP |
| | | |
| G | EXTRAIT DE LENTINUS selon l'exemple 1 | 0,2 à 10 |
| | | |
| I | PARFUM | QS |

| Mode opératoire : |
|---|
| Faire chauffer les phases A et B vers 75°C puis ajouter dans les phases B1, B2 et B3. Ajouter B1+2+3 dans la phase A et ajouter la phase C. Aux alentours d'une température à 60°C, ajouter la phase D et à une température inférieure à 50°C, ajouter les autres phases. |

**Exemple 12 : Composition cosmétique à base de lentinus pour une crème**

| Phase | Matière première / Nom commercial | % |
|---|---|---|
| A1 | GLYCERYL MYRISTATE | 5,0 |
| | DIISOPROPYL SEBACATE | 5,0 |
| | | |
| A2 | STEARYL DIMETHICONE | 1,0 |
| | | |
| A3 | PALMOTENE | 0,5 |
| | | |
| B1 | EAU OSMOSEE | QSP 100 |
| | PROPYLENE GLYCOL | 1,0 |
| | CONSERVATEUR | QS |
| | | |
| B2 | POTASSIUM CETYL PHOSPHATE | 4,0 |
| | | |
| C | ACIDE CITRIQUE (SOLUTION A 10%) | QSP pH=6.5 |
| D | EXTRAIT DE LENTINUS | 0,25 à 10 |
| | | |
| F | PARFUM | QS |

| Mode opératoire : |
|---|
| Faire chauffer les phases A1 et B1 vers 75°C. Ajouter les phases A2 et A3 dans A1 et B2 dans B1, agiter puis ajouter la phase B dans la phase A- Ajouter la phase C à une température inférieure à 60°C puis la phase D. A une température inférieure à 50°C, ajouter les autres phases. |

## Revendications

1. Extrait polysaccharidique issu de champignon du genre Lentinus **caractérisé par le fait qu'**il comprend des monosaccharides, des polysaccharides, et en poids, de 2 à 8 % de tréhalose, de 1 à 4 % de glucose et de 30 à 60 % d'alditols par rapport au poids total de l'extrait sec et qu'il est susceptible d'être obtenu par le procédé tel que défini dans l'une quelconque des revendications 7 et 8.

2. Extrait polysaccharidique selon la revendication 1, **caractérisé par le fait qu'**il comprend en poids, de 2 à 6 % de tréhalose, de 2 à 3 % de glucose et de 45 à 55 % d'alditols par rapport au poids total de l'extrait sec.

3. Extrait polysaccharidique selon la revendication 1 ou 2, **caractérisé par le fait qu'**il comprend en poids de 3 à 5 % de tréhalose, de 2 à 3 % de glucose et de 45 à 50 % d'alditols par rapport au poids total de l'extrait sec.

4. Extrait polysaccharidique selon l'une quelconque des revendications 1 à 3, **caractérisé par le fait que** les alditols comprennent de l'arabinitol et du mannitol.

5. Extrait polysaccharidique selon l'une quelconque des revendications 1 à 4 **caractérisé par le fait qu'**il se présente sous forme de poudre.

6. Extrait polysaccharidique selon la revendication 1, défini par les analyses de spectrométries de masse des figures 1 et 2.

7. Procédé de préparation d'un extrait polysacharidique selon l'une quelconque des revendications 1 à 6 **caractérisé par le fait que** l'on procède, à partir de champignons Lentinus Edodes ou Shii-take, à une ou plusieurs extractions solide/liquide à l'eau bouillante, l'extrait liquide est précipité par de l'éthanol, centrifugé, filtré, puis le précipité est ensuite séché pour obtenir l'extrait polysaccharidique.

8. Procédé selon la revendication 7, dans lequel les champignons sont séchés.

9. Composition nutraceutique **caractérisée par le fait qu'**elle contient un extrait polysaccharidique tel que défini dans l'une quelconque des revendications 1 à 6.

10. Utilisation d'un extrait polysaccharidique tel que défini dans l'une quelconque des revendications 1 à 6 pour la préparation d'un produit nutraceutique pour combattre le vieillissement de la peau et des muqueuses causé par les agressions extérieures et ses conséquences

11. Composition cosmétique **caractérisée par le fait qu'**elle contient, dans un milieu cosmétiquement acceptable, un extrait polysaccharidique tel que défini dans l'une quelconque des revendications 1 à 6.

12. Utilisation cosmétique de l'extrait polysaccharidique tel que défini dans l'une quelconque des revendications 1 à 6 pour combattre le vieillissement de la peau et des muqueuses causé par les agressions extérieures.

13. Extrait polysaccharidique selon l'une quelconque des revendications 1 à 6 pour son utilisation comme médicament.

14. Utilisation d'un extrait polysaccharidique selon l'une quelconque des revendications 1 à 6 pour la préparation d'un médicament destiné à traiter les dermatoses.

## Patentansprüche

1. Polysaccharidextrakt aus einem Pilz der Gattung Lentinus, **dadurch gekennzeichnet, dass** er Monosaccharide, Polysaccharide sowie 2 bis 8 Gew.-% Trehalose, 1 bis 4 Gew.-% Glucose und 30 bis 60 Gew.-% Aldite in Bezug auf das Gesamtgewicht des trockenen Extrakts umfasst und dadurch, dass er nach dem Verfahren wie in einem der Ansprüche 7 und 8 definiert erhältlich ist.

2. Polysaccharidextrakt nach Anspruch 1, **dadurch gekennzeichnet, dass** er 2 bis 6 Gew.-% Trehalose, 2 bis 3 Gew.-% Glucose und 45 bis 55 Gew.-% Aldite in Bezug auf das Gesamtgewicht des trockenen Extrakts umfasst.

3. Polysaccharidextrakt nach Anspruch 1 oder 2, **dadurch gekennzeichnet, dass** er 3 bis 5 Gew.-% Trehalose, 2 bis 3 Gew.-% Glucose und 45 bis 50 Gew.-% Aldite in Bezug auf das Gesamtgewicht des trockenen Extrakts umfasst.

4. Polysaccharidextrakt nach einem der Ansprüche 1 bis 3, **dadurch gekennzeichnet, dass** die Aldite Arabinit und Mannit umfassen.

5. Polysaccharidextrakt nach einem der Ansprüche 1 bis 4, **dadurch gekennzeichnet, dass** er in Pulverform vorliegt.

6. Polysaccharidextrakt nach Anspruch 1, der durch die massenspektrometrischen Analysen der Figuren 1 und 2 definiert ist.

7. Verfahren zur Herstellung eines Polysaccharidextrakts nach einem der Ansprüche 1 bis 6, **dadurch gekennzeichnet, dass** man mit den Pilzen Lentinus edodes oder Shiitake eine oder mehrere Fest-Flüssig-Extraktionen mit kochendem Wasser vornimmt, der flüssige Extrakt mit Ethanol gefällt und dann zentrifugiert und filtriert wird und der Niederschlag dann getrocknet wird, wodurch man zu dem Polysaccharidextrakt gelangt.

8. Verfahren nach Anspruch 7, wobei die Pilze getrocknet werden.

9. Nutrazeutische Zusammensetzung, **dadurch gekennzeichnet, dass** sie einen Polysaccharidextrakt wie in einem der Ansprüche 1 bis 6 definiert enthält.

10. Verwendung eines Polysaccharidextrakts wie in einem der Ansprüche 1 bis 6 definiert für die Herstellung eines nutrazeutischen Produkts zum Bekämpfen der von äußeren Aggressionen verursachten Haut- und Schleimhautalterung und ihrer Folgeerscheinungen.

11. Kosmetikzusammensetzung, **dadurch gekennzeichnet, dass** sie einen Polysaccharidextrakt wie in einem der Ansprüche 1 bis 6 definiert in einem kosmetisch unbedenklichen Medium enthält.

12. Kosmetische Verwendung des Polysaccharidextrakts wie in einem der Ansprüche 1 bis 6 definiert zum Bekämpfen der von äußeren Aggressionen verursachten Haut- und Schleimhautalterung.

13. Polysaccharidextrakt nach einem der Ansprüche 1 bis 6 zur Verwendung als Arzneimittel.

14. Verwendung eines Polysaccharidextrakts nach einem der Ansprüche 1 bis 6 zur Herstellung eines Arzneimittels für die Behandlung von Dermatosen.

## Claims

1. Polysaccharide extract derived from a mushroom of the Lentinus genus, **characterized in that** it comprises monosaccharides, polysaccharides, and by weight, from 2% to 8% of trehalose, from 1% to 4% of glucose and from 30% to 60% of alditols, relative to the total weight of the dry extract and **in that** it can be obtained by means of the method as defined in either one of Claims 7 and 8.

2. Polysaccharide extract according to Claim 1, **characterized in that** it comprises, by weight, from 2% to 6% of trehalose, from 2% to 3% of glucose and from 45% to 55% of alditols, relative to the total weight of the dry extract.

3. Polysaccharide extract according to Claim 1 or 2, **characterized in that** it comprises, by weight, from 3% to 5% of trehalose, from 2% to 3% of glucose and from 45% to 50% of alditols, relative to the total weight of the dry extract.

4. Polysaccharide extract according to any one of Claims 1 to 3, **characterized in that** the alditols comprise arabinitol and mannitol.

5. Polysaccharide extract according to any one of Claims 1 to 4, **characterized in that** it is in powder form.

6. Polysaccharide extract according to Claim 1, defined by the mass spectrometry analyses of Figures 1 and 2.

7. Method for preparing a polysaccharide extract according to any one of Claims 1 to 6, **characterized in that** one or more solid/liquid extractions are carried out with boiling water on Lentinus edodes or shii-take mushrooms, the liquid extract is precipitated with ethanol, centrifuged and filtered, and then the precipitate is subsequently dried so as to obtain the polysaccharide extract.

8. Method according to Claim 7, in which the mushrooms are dried.

9. Nutraceutical composition, **characterized in that** it contains a polysaccharide extract as defined in any one of Claims 1 to 6.

10. Use of a polysaccharide extract as defined in any one of Claims 1 to 6, for the preparation of a nutraceutical product for combating ageing of the skin and of the mucous membranes caused by outside attacks, and its consequences.

11. Cosmetic composition, **characterized in that** it contains, in a cosmetically acceptable medium, a polysaccharide extract as defined in any one of Claims 1 to 6.

12. Cosmetic use of the polysaccharide extract as defined in any one of Claims 1 to 6, for combating ageing of the skin and of the mucous membranes caused by outside attacks.

13. Polysaccharide extract according to any one of Claims 1 to 6, for its use as a medicament.

14. Use of a polysaccharide extract according to any one of Claims 1 to 6, for the preparation of a medicament for use in treating dermatosis.
